# EUROPÄISCHE PATENTANMELDUNG

(11) **EP 1 845 355 A2**
(43) Veröffentlichungstag der Anmeldung: **17.10.2007**
(21) Anmeldenummer: 07105956.2
(22) Anmeldetag: 11.04.2007
(51) Int. Cl.: G01N 1/18, G01N 35/04, B01L 9/00, B62B 1/00, B62B 3/00, G01N 33/18

(54) **Aufnahmebehälter für einen Probennehmer oder einen Analysator**

(30) Priorität: 11.04.2006 DE 102006017374
(71) Anmelder: Endress+Hauser Conducta Gesellschaft für Mess- und Regeltechnik mbH+Co. KG, 70839 Gerlingen (DE)
(72) Erfinder: Wandrei, Norbert, 70599, Stuttgart (DE); Steuerwald, Ralf, 73642, Welzheim (DE)
(74) Vertreter: Andres, Angelika Maria

(57) **Zusammenfassung**

Die Erfindung betrifft einen Aufnahmebehälter (1) für einen Probennehmer oder einen Analysator zur Aufnahme von zumindest einem Behältnis (2) zur Aufbewahrung einer flüssigen Probenmenge, wobei derAufnahmebehälter (1) so ausgestaltet ist, dass er in dem Probennehmer oder Analysator positionier-bar und aus dem Probennehmer entnehmbar ist, wobei im unteren Bereich (6) des Aufnahmebehälters (1) zumindest eine Transportrolle (3) bzw. ein Transportrad (3) zum Transport des Aufnahmebehälters (1) vorgesehen ist und wobei im oberen Bereich (7) des Aufnahmebehälters (1) zumindest ein Griffteil (5) vorgesehen ist.

## Beschreibung

Die Erfindung betrifft einen Aufnahmebehälter für einen Probennehmer oder einen Analysator zur Aufnahme von zumindest einem Behältnis zur Aufbewahrung einer flüssigen Probenmenge.

Zur kontinuierlichen Überwachung und ggf. Vorort-Analyse von flüssigen Proben wird üblicherweise ein Probennehmer in der Nähe der Proben-entnahmestelle plaziert. Über einen automatischen Ansaug- und Abfüll-mechanismus werden in zeitlich vorgegebenen Abständen flüssige Proben mittels einer Dosierpumpe angesaugt und in Probenflaschen oder Probenkanister eingefüllt. Zwecks Transport und Aufbewahrung sind die Probenflaschen- oder Kanister in einem Aufnahmebehälter angeordnet. Üblicherweise hat der Aufnahmebehälter die Form eines Flaschenkorbs mit einem Tragegriff bzw. mit Tragegriffen. Dieser Flaschenkorb soll den Transport bzw. den Austausch der Flaschen oder Kanister in dem Probennehmer bzw. in dem Analysator erleichtern.

Bekannt geworden sind einteilige Flaschenkörbe mit Tragegriffen. Diese zeichnen sich dadurch aus, dass die Flaschen in einem möglichst begrenzten Raumbereich sehr kompakt angeordnet werden können. Nachteilig ist jedoch das relativ hohe Gewicht des Flaschenkorbs bzw. Aufnahmebehälters, wenn die Flaschen bzw. die Kanister mit dem Fluid gefüllt sind. Zur Reduzierung des Gewichts und damit zur Erleichterung des Transports ist es weiterhin bekannt, den Aufnahmebehälter zweiteilig auszuführen. Auf diese Weise lässt sich das Gewicht des Aufnahmebehälters halbieren. Die erleichterte Transport geht dann jedoch auf Kosten der kompakten Bauweise des Probennehmers bzw. Analysators. Als Folge hiervon benötigt der Probennehmer / Analysator eine größere Stellfläche, was in vielen Anwendungsfällen problematisch ist, z.B. dann wenn der Probennehmer in einem Gully plaziert wird, um Abwasserproben zu sammeln.

Unter der Bezeichnung LIQUIPORT 2000 wird von der Anmelderin ein tragbarer Probennehmer angeboten und vertrieben. Eine sehr vorteilhafte Ausgestaltung dieses Probennehmers ist in der DE 102 52 158 B4 beschrieben. In dieser Patentschrift wird ein Speicher- oder Transportbehälter für einen Probennehmer vorgestellt, wobei der Speicherbehälter so ausge-staltet ist, dass er zum Transportieren der Flaschen mit einem Deckel versehen wird. Hierdurch entfällt das Umräumen der Probenflaschen in einen entsprechenden Transportbehälter bzw. in einen entsprechenden Flaschenkorb und der mit gefüllten Flaschen befüllte Transportbehälter wird durch einen mit leeren Flaschen befüllten Transportbehälter ersetzt. Auch für einen derartigen Probennehmer mit einem austauschbaren und als Aufnahmebehälter ausgebildeten Unterteil ist die vorliegende Erfindung bestens geeignet.

Der Erfindung liegt die Aufgabe zugrunde, einen Probennehmer / Analysator bzw. einen Aufnahmebehälter für einen Probennehmer / Analysator vorzuschlagen, der sich trotz kompakter Bauweise auf einfache Art und Weise transportieren lässt.

Die Aufgabe wird dadurch gelöst, dass der Aufnahmebehälter so ausgestaltet ist, dass er von dem Probennehmer oder Analysator trennbar ist bzw. in dem Probennehmer positionierbar bzw. aus dem Probennehmer entnehmbar ist, dass im unteren Bereich des Aufnahmebehälters zumindest eine Transport-rolle bzw. ein Transportrad zum Transport des Aufnahmebehälters vorge-sehen ist und dass im oberen Bereich des Aufnahmebehälters zumindest ein Griffteil vorgesehen ist. Bevorzugt handelt es sich bei dem Griffteil um eine Deichsel oder um eine Schnur. Bei dem Behältnis kann es sich um zumindest eine Flasche oder um zumindest einen Kanister handeln.

Gemäß einer vorteilhaften Ausgestaltung ist zumindest eine Transportrolle am bzw. im unteren Bereich des erfindungsgemäßen Aufnahmebehälters derart angebracht, dass der Aufnahmebehälter auf der Transportrolle bewegbar ist, sobald der Aufnahmebehälter eine Neigung um einen vorgegebenen Winkel zur Normalen erfährt.

Weiterhin ist vorgesehen, dass der Aufnahmebehälter eine rechteckige Form aufweist. Selbstverständlich kann er jedoch auch die Form des Unterteils aufweisen, wie es in der DE 102 52 158 B4 beschrieben ist.

Bei einem im wesentlichen rechteckförmigen Aufnahmebehälter sind Transporträder bzw. Transportrollen vorgesehen, die an gegenüberliegenden, in Bewegungsrichtung gesehen hinten liegenden Eckbereichen des Aufnahmebehälters befestigt bzw. befestigbar sind.

Eine alternative Ausgestaltung sieht vier Transporträder vor, welche in den Eckbereichen des Aufnahmebehälters befestigt bzw. befestigbar sind.

Eine vorteilhafte Ausführungsform des erfindungsgemäßen Aufnahme-behälters schlägt vor, dass die Transporträder bzw. die zumindest eine Transportrolle an dem Aufnahmebehälter befestigbar bzw. von dem Aufnahmebehälter entfernbar sind. Hierdurch wird ggf. eine Positionierung des Aufnahmebehälters in dem Gehäuse des Probennehmers erleichtert.

Gleiches ist auch im Hinblick auf das Griffteil vorgesehen.

Die Erfindung wird anhand der nachfolgenden Figuren näher erläutert. Es zeigt:

Fig. 1: eine perspektivische Darstellung einer ersten Ausgestaltung des erfindungsgemäßen Aufnahmebehälters und

Fig. 2: eine perspektivische Darstellung einer zweiten Ausgestaltung des erfindungsgemäßen Aufnahmebhälters.

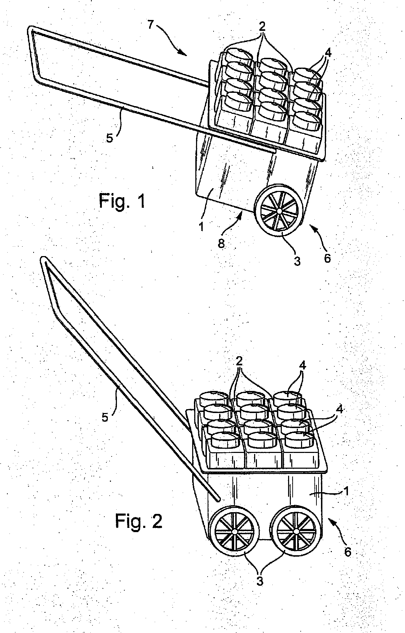

Fig. 1 zeigt eine perspektivische Darstellung einer ersten Ausgestaltung des erfindungsgemäßen Aufnahmebhälters 1. Der Aufnahmebehälter 1 hat die Form einer im oberen Bereich offenen rechteckförmigen Kiste. In dem Aufnahmebehälter 1 sind im dargestellten Fall zwölf mit jeweils einem Deckel 4 verschließbare Flaschen 2 gelagert. Zum erleichterten Transport der mit Probenflüssigkeit gefüllten Flaschen 2 ist der Aufnahmebehälter 1 fahrbar bzw. rollbar ausgestaltet. Hierzu sind - je nach Bewegungsrichtung - bei der in Fig. 1 vorgeschlagenen Lösung zwei Transporträder 3 im hinteren bzw. im vorderen unteren Bereich 6 des Aufnahmebehälters 1 vorgesehen. Mittels des Griffteils 5, bei dem es sich im dargestellten Fall um eine Deichsel handelt, wird der Aufnahmebehälter 1 an den Zielort gezogen oder gedrückt.

Bei der in Fig. 2 gezeigten Lösung sind im unteren Bereich 6 des Aufnahme-behälters 1 vier Transporträder 3 vorgesehen. Die Transporträder 3 sind in den vier Eckbereichen des Aufnahmebehälters 1 vorgesehen. Bevorzugt sind die Transporträder 3 und ggf. auch die Deichsel 5 bei Bedarf an dem Aufnahmebehälter 1 adaptierbar. Hierzu ist beispielsweise eine die Transporträder 3 tragende Achse über eine entsprechende Lagerung an der Unterseite des Aufnahmebehälters 1 befestigbar.

## Patentansprüche

1. Aufnahmebehälter (1) für einen Probennehmer oder einen Analysator zur Aufnahme von zumindest einem Behältnis (2) zur Aufbewahrung einer flüssigen Probenmenge, wobei der Aufnahmebehälter (1) so ausgestaltet ist, dass er in dem Probennehmer oder Analysator positionierbar und aus dem Probennehmer entnehmbar ist, wobei im unteren Bereich (6) des Aufnahmebehälters (1) zumindest eine Transportrolle (3) bzw. ein Transportrad (3) zum Transport des Aufnahme-behälters (1) vorgesehen ist und wobei im oberen Bereich (7) des Aufnahmebehälters (1) zumindest ein Griffteil (5) vorgesehen ist.

2. Aufnahmebehälter nach Anspruch 1, wobei es sich bei dem Griffteil (5) um eine Deichsel oder um eine Schnur handelt.

3. Aufnahmebehälter nach Anspruch 1, wobei die zumindest eine Transportrolle am unteren Bereich des Aufnahmebehälters derart angebracht ist, dass der Aufnahmebehälter auf der Transportrolle bewegbar ist, sobald der Aufnahmebehälter eine Neigung um einen vorgegebenen Winkel zur Normalen erfährt.

4. Aufnahmebehälter nach Anspruch 1, 2 oder 3, wobei der Aufnahmebehälter (1) eine im wesentlichen rechteckige Grundfläche (8) Form aufweist.

5. Aufnahmebehälter nach Anspruch 1, 3 oder 4, wobei Transporträder (3) bzw. Transportrollen (3) vorgesehen sind, die an gegenüberliegenden, in Bewegungsrichtung gesehen hinten liegenden Eckbereichen des Aufnahmebehälters (1) befestigt bzw. befestigbar sind.

6. Aufnahmebehälter nach Anspruch 1 oder 4, wobei vier Transporträder (3) vorgesehen sind, in den Eckbereichen des Aufnahmebehälters (1) befestigt bzw. befestigbar sind.

7. Aufnahmebehälter nach einem oder mehreren der vorhergehenden Ansprüche, wobei ein Mechanismus, vorzugsweise ein Clipsmechanismus, vorgesehen ist, über den die Transporträder (3) bzw. die zumindest eine Transportrolle (3) an dem Aufnahmebehälter (1) befestigbar bzw. von dem Aufnahmebehälter (1) entfernbar sind.

8. Aufnahmebehälter nach Anspruch 1, wobei es sich bei dem Behältnis (2) um zumindest eine Flasche oder einen Kanister handelt.
